# EUROPEAN PATENT APPLICATION

(11) **EP 3 287 095 A1**
(43) Date of publication of application: **28.02.2018**
(21) Application number: 17187487.8
(22) Date of filing: 23.08.2017
(51) Int. Cl.: A61C 8/00, A61C 9/00, A61B 90/00

(54) **DENTAL IMPLANT WITH INTEGRATED SCAN BODY**

(30) Priority: 24.08.2016 IL 24747316
(71) Applicant: Musheev, Ilya, Holon (IL)
(72) Inventor: Musheev, Ilya, Holon (IL)
(74) Representative: Anohins, Vladimirs

(57) **Abstract**

The present invention is a dental implant with an integrated scan body located between the apical and coronal portions of the implant. The scan body has a geometry permitting unequivocal determination of its position relatively to the position of neighboring teeth, from different scanning directions.

## Description

### BACKGROUND

### 1. Technical Field

The present invention relates to the field of devices for dental implantation. More specifically, the invention relates to a dental implant with an integrated scan body.

### 2. Related Art

In the field of artificial tooth replacement where teeth have to be replaced, the corresponding dental prostheses are usually fixed in a patient's mouth via a dental implant that is fixed in the bone of a patient's jaw. Whereas there is usually sufficient space for placing a dental implant between two neighboring teeth, the situation is more complicated for the corresponding dental prosthesis. In order to achieve a qualitatively and aesthetically good result, a dental prosthesis has to fit almost perfectly between neighboring teeth. Besides the final location of a dental prosthesis within an oral environment, it is also beneficial to ensure that a dental prosthesis can actually be inserted between two neighboring teeth and, at the same time, can be connected with the implant.

In order to achieve the above-mentioned tasks and requirements, a possible solution is to determine the position and the orientation of a dental implant with respect to neighboring teeth and the gingiva between these neighboring teeth with precision. Typically, this determination has to be performed in a situation where the implant is not directly visible.

In the prior art, the position and orientation of a dental implant is determined by attaching a scan body to the implant, determining the position and orientation of the scan body and determining the position and orientation of the implant relative to the position and orientation of the scan body. In some cases, the scanning procedure is carried out with the help of a model that represents the situation of a patient's mouth or, in other cases, the procedure is carried out directly in a patient's mouth. In other cases the scanning procedure is done extra-orally and a computerized model that represents the situation of a patient's mouth is created.

### SUMMARY OF INVENTION

According to one aspect of the present invention there is provided a dental implant with an integrated scan body that has a symmetrical or an asymmetrical geometry permitting unequivocal determination of its position relatively to a position of neighboring teeth, from different scanning directions.

Another aspect of the present invention is a method for determining a position and an orientation of a dental implant. The method for determining a position and an orientation of a dental implant is a combination of an extra-oral scanning procedure (X-ray based or other) and a reconstruction procedure. During the scanning procedure, the surface of a scan body is scanned and a virtual scan body or scan body surface is created by determining a plurality of data points that correspond to positions of points that are located on the surface of the scan body. During the reconstruction a virtual scan body is associated with a physical scan body by associating virtual scan body elements with physical scan body elements.

### BRIEF DESCRIPTION OF THE DRAWINGS

Additional advantages and features of the invention are revealed in the claims and the following description of a preferred exemplary embodiment of the invention that is explained with reference to the drawings, in which:
**FIG. 1** is a perspective view of a dental implant with a cylindrical scan body;
**FIG. 2** is a perspective view of a dental implant with a hexagonal scan body;
**FIG. 3** is a perspective view of a dental implant with a scan body having at least one groove;
**FIG. 4** is a perspective view of a dental implant with a quadrilateral scan body.

### DETAILED DESCRIPTION

In the following detailed description of the invention, numerous specific details are set forth in order to provide a thorough understanding of the invention. However, it will be obvious to one skilled in the art that the invention may be practiced without these specific details. In other instances, well known methods, procedures, components, and elements have not been described in detail so as not to unnecessarily obscure aspects of the invention.

It will be readily understood that the components of the present invention, as generally described and illustrated in the figures, may be arranged and designed in a wide variety of different configurations. Thus, the following more detailed description of the embodiments of the apparatus and methods of the present invention, as represented in the figures, is not intended to limit the scope of the invention, but is merely representative of selected embodiments of the invention.

The present invention is an implant with an integrated scan body. The scan body may have a polygonal or a cylindrical (or any other) shape, and it has either a symmetrical or an asymmetrical geometry (in relation to its longitudinal central axis). The shape and/or geometry of the scan body makes it possible (by scanning it from different directions) to determine the exact position and the orientation of the scan body.

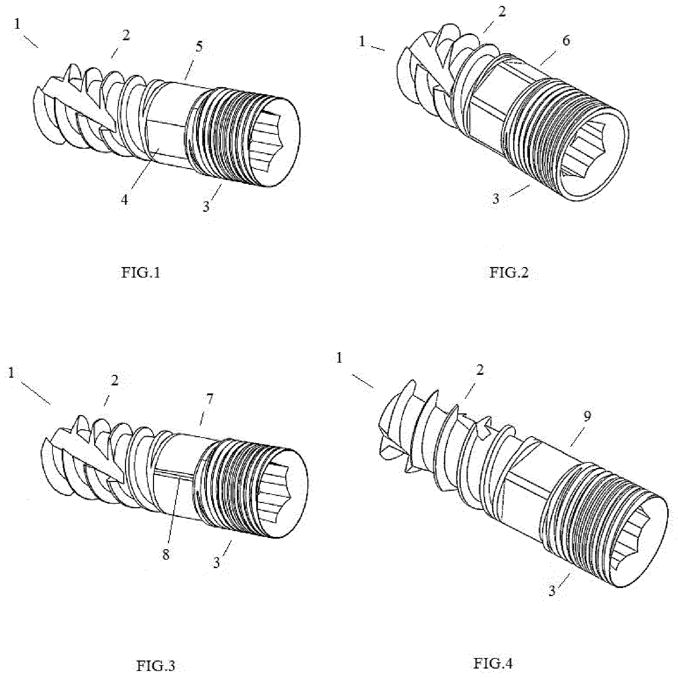

Fig. 1 illustrates an embodiment of a dental implant **1** having a threaded apical portion **2** and a threaded coronal portion **3.** The implant **1** includes a scan body **5** located between said threaded apical portion **2** and the coronal portion **3.** The scan body **5** has a form of a cylinder with an at least one cut off segment **4.** The scan body **5** has either a symmetrical or an asymmetrical geometry, permitting unambiguous determination of its position relatively to the position of the neighboring teeth and/or jaw bone, from different scanning directions.

Fig. 2 illustrates another embodiment of a scan body of the present invention. In this embodiment, a scan body is a hexagonal scan body 6 that may include chamfered edges. The hexagonal scan body **6** has either a symmetrical or an asymmetrical geometry, permitting unambiguous determination of its position relatively to the position of the neighboring teeth and/or jaw bone, from different scanning directions.

Fig. 3 illustrates yet another embodiment of a scan body of the present invention. In this embodiment a scan body **7** has a form of a cylinder with an at least one groove **8.** The scan body **7** has either a symmetrical or an asymmetrical geometry, permitting unambiguous determination of its position relatively to the position of the neighboring teeth and/or jaw bone, from different scanning directions.

Fig. 4 illustrates yet another embodiment of a scan body of the present invention. In this embodiment a scan body is a quadrilateral scan body **9** that may include chamfered edges. The quadrilateral scan body **9** has either symmetrical or asymmetrical geometry, permitting unambiguous determination of its position relatively to the position of the neighboring teeth and/or jaw bone, from different scanning directions.

Another aspect of the present invention is a method for determining a position and an orientation of a dental implant.

In one embodiment, a method for determining a position and an orientation of a dental implant is a combination of an extra-oral scanning procedure (X-ray based or other) and a reconstruction procedure. During the scanning procedure, a surface of a scan body is scanned and a virtual scan body or a scan body surface is created by determining a plurality of data points that correspond to positions of points that are located on the surface of the physical scan body.

In another embodiment, an already existing data set, having a plurality of data points corresponding to positions of points that are located on the surface of a scan body, is loaded in order to perform the reconstruction of the position and orientation of a dental implant.

During the reconstruction phase, these data points can be used for reconstructing at least three planes. From the reconstructed planes, intersection information is determined. The intersection information comprises straight intersection lines where at least two planes intersect each other and/or intersection points where at least three planes intersect each other or where a straight intersection line intersects a plane or where at least two straight intersection lines intersect each other. Using the above-mentioned reconstructed intersection information, the position and orientation of an implant can be determined. This determination can be based on only part of the reconstructed intersection information or it can be based on all available reconstructed intersection information.

It should be noted that the scanning points on the surface of a scan body do not necessarily have to correspond to corners or edges. Any point within a surface area can be used for the above-mentioned reconstruction procedure, which simplifies the scanning procedure.

In yet another method of the present invention, the determination of the position and orientation of a dental implant comprises associating reconstructed geometrical elements with physical geometrical elements of a scan body. For instance, reconstructed planes can be associated with physical plane areas of the surface of the scan body. Alternatively, or in addition, reconstructed straight intersection lines can be associated with physical edges of the scan body and/or reconstructed intersection points can be associated with physical corners of the scan body. It is noted that physical corners (and also edges) are not perfect corners in a mathematical sense, but are rounded. It should be understood that the reconstructed geometrical objects correspond to physical geometrical objects of a part of the scan body that actually has been scanned.

In yet another embodiment of the present invention, the method for determining the position and orientation of a dental implant further comprises the generation of a digital/virtual model of at least a part of an implant inside the mouth of a patient. The digital/virtual model is preferably three-dimensional so that the model can be viewed from different angles, which is useful to study the insertion of a dental prosthesis, for instance.

The digital/virtual model can further reflect information about neighboring teeth and the gingiva in the neighborhood of the implant.

Having thus described the present invention by reference to certain of its preferred embodiments, it is noted that the embodiments disclosed are illustrative rather than limiting in nature and that a wide range of variations, modifications, changes, and substitutions are contemplated in the foregoing disclosure and, in some instances, some features of the present invention may be employed without a corresponding use of the other features.

## Claims

1. A dental implant comprising: a threaded apical portion; a threaded coronal portion; a scan body, located between said apical and coronal portions, and wherein said scan body has a geometry permitting unequivocal determination of its position relatively to a position of neighboring teeth, from different scanning directions.

2. The dental implant of claim 1, wherein said scan body has a symmetrical geometry in relation to its longitudinal central axis.

3. The dental implant of claim 1, wherein said scan body has an asymmetrical geometry in relation to its central axis.

4. The dental implant of claim 1, wherein said scan body has a cylindrical geometry with an at least one cut off segment.

5. The dental implant of claim 1, wherein said scan body has at least one groove.

6. The dental implant of claim 1, wherein said scan body has a quadrilateral geometry.

7. The dental implant of claim 1, wherein said scan body has a polygonal geometry.

8. The dental implant of claim 1, wherein said scan body has a hexagonal geometry.

9. A method for determining a position and an orientation of a dental implant in a jaw bone of a patient, the method comprising: performing an extra-oral, X-ray based, scanning procedure of an implant having an integrated scan body, while said implant is positioned in a jaw bone; creating a virtual scan body by determining a plurality of data points that correspond to positions of points that are located on a surface of the scan body; associating the virtual scan body with a physical scan body by associating virtual scan body elements with physical scan body elements.
